# EUROPÄISCHE PATENTANMELDUNG

(11) **EP 1 775 303 A1**
(43) Veröffentlichungstag der Anmeldung: **18.04.2007**
(21) Anmeldenummer: 06021646.2
(22) Anmeldetag: 16.10.2006
(51) Int. Cl.: C07H 9/04, C07H 11/00, A61K 31/70, A61P 25/08

(54) **Topiramat und pharmazeutische Formulierungen davon**

(30) Priorität: 17.10.2005 DE 202005016250 U
(71) Anmelder: Helm AG, 20097 Hamburg (DE)
(72) Erfinder: Generlich, Olaf, 22175 Hamburg (DE); Glänzer, Klaus, Dr., 22337 Hamburg (DE)
(74) Vertreter: Teipel, Stephan

(57) **Zusammenfassung**

Die vorliegende Erfindung betrifft Topiramat sowie pharmazeutische Formulierungen, die diesen Wirkstoff enthalten.

## Beschreibung

Die vorliegende Erfindung betrifft Topiramat sowie pharmazeutische Formulierungen, die diesen Wirkstoff enthalten.

Topiramat (2,3:4,5-Bis-*O*-(1-methylethyliden)-β-D-fructopyranosesulfamat) der allgemeinen Formel 1: 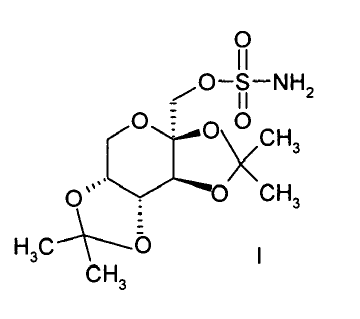 ist als Antiepileptikum bekannt. Die Verbindung, die zur Klasse der Sulfamoylsubstituierten Saccharide gehört, wird in EP-A-0 138 441 offenbart.

Verfahren zur Synthese von Topiramat sind beispielsweise in EP-A-0 533 483, WO 03/097656 sowie der noch unveröffentlichten Europäischen Patentanmeldung Nr. 04.019684.2 beschrieben.

Topiramat wird handelsüblich in Form von Tabletten angeboten, die u.a. unter dem Handelsnamen Topamax zur oralen Verabreichung mit 25 mg, 50 mg, 100 mg, 200 mg Wirkstoff angeboten werden. Die WO 98/00130 offenbart, dass diese Tabletten wasserfreie Lactose, pregelatinisierte Stärke, mikrokristalline Cellulose, Natriumstärkeglycolat, Magnesiumstearat, gereinigtes Wasser, Carnaubawachs, Hydroxypropylmethylcellulose, Titandioxid, Polyethylenglycol, synthetisches Eisenoxid und Polysorbat 80 als inaktive Bestandteile enthalten.

Die WO 01/89445 beschreibt, dass Topiramat-Tabletten in Gegenwart von Feuchtigkeit und Wärme instabil sind, was zu sichtbaren braunen bis schwarzen Verfärbungen sowie zur Bildung von Sulfationen führt. Je mehr freies Wasser in den Tabletten enthalten ist, desto schneller treten Abbaureaktionen auf, die sich durch farbliche Veränderungen bemerkbar machen. Darüber hinaus nehmen Topamax-Tabletten sehr schnell Feuchtigkeit auf. Beispielsweise erhöht sich das Tablettengewicht gemäß WO 01/89445 bei 60 % relativer Feuchtigkeit (r.F.) nach zwei Stunden um ca. 1,2 %.

Dieses Stabilitätsproblem von Topiramat-Tabletten wurde ursprünglich durch Verpacken der Tabletten in HDPE-Flaschen, die mit einem Trockenmittel versehen wurden, gelöst. Alternativ wurde vorgeschlagen, die Tabletten in Blisterverpackungen anzubieten, die ebenfalls mit einem Trockenmittel versehen sein sollten. Um die Anwesenheit eines Trockenmittels zu vermeiden, schlägt die WO 01/89445 vor, die Topiramat-Tabletten vor dem Einblistern auf einen Restgehalt an freiem Wasser zwischen 0,4 % und 1,4 % zu trocknen. Beide Methoden weisen den Nachteil auf, dass sie zeit- und kostenintensiv sind.

Es besteht daher weiterhin ein Bedürfnis nach pharmazeutischen Formulierungen für Topiramat, die auch ohne aufwendige Verpackungen oder Vorbehandlungen vor dem Verpacken stabil sind. Eine Aufgabe der vorliegenden Erfindung besteht somit darin, Topiramat in einer Form zur Verfügung zu stellen, die auch bei längerer Lagerung und selbst unter erhöhten Temperaturen lagerstabil ist und sich somit zur Herstellung pharmazeutischer Formulierungen besonders eignet.

Es wurde nun überraschend gefunden, dass Topiramat praktisch nicht mehr hygroskopisch ist, wenn es in besonders reiner Form und mit einer kleinen Korngröße vorliegt. Die vorliegende Erfindung betrifft somit Topiramat, das dadurch gekennzeichnet ist, dass es in hoch reiner Form vorliegt und mindestens 90 % der Teilchen eine Korngröße von ≤ 250 µm aufweisen.

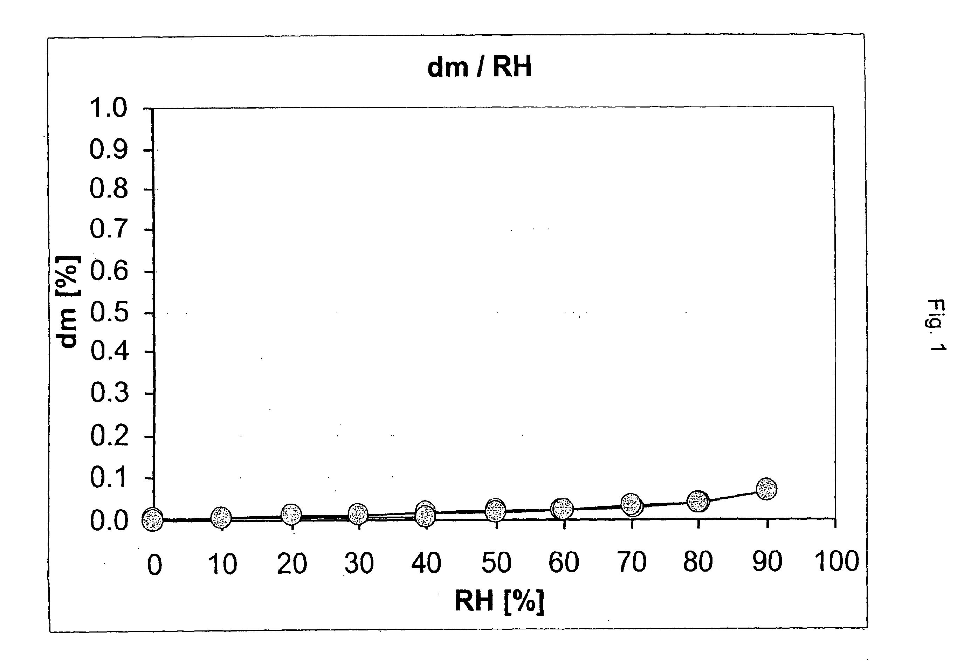

Es hat sich gezeigt, dass hoch reines Topiramat, das in der angegebenen Korngröße vorliegt, im wesentlichen nicht hygroskopisch ist. So nimmt der reine Wirkstoff bei 25°C und 90 % r.F. weniger als 0,1 % Wasser auf, wie der in Fig. 1 abgebildeten Sorptionsisotherme zu entnehmen ist. Eine Lagerung selbst bei 80°C über einen Monat führt zu keinen farblichen Veränderungen. Das erfindungsgemäße Topiramat eignet sich somit in besonderer Weise zur Herstellung pharmazeutischer Formulierungen.

Unter Topiramat in hoch reiner Form wird vorliegend ein Wirkstoff verstanden, der zu mindestens 99 Gew.-% Topiramat enthält. Vorzugsweise enthält der Wirkstoff höchstens 0,5 Gew.-%, insbesondere höchstens 0,2 Gew.-% Verunreinigungen, wobei Wasser nicht als Verunreinigung gerechnet wird.

Die Korngröße des Wirkstoffs wird erfindungsgemäß so gewählt, dass mindestens 90 % der Teilchen eine Korngröße von ≤ 250 µm aufweisen. Vorzugsweise weisen mindestens 90 % der Teilchen eine Korngröße von ≤ 200 µm auf. Die mittlere Korngröße sollte ≤ 150 µm, vorzugsweise ≤ 120 µm und besonders bevorzugt im Bereich von 30-80 µm sein. Geeignete Verfahren zur Korngrößeneinstellung sind dem Fachmann bekannt und können von diesem aufgrund seines Fachwissens leicht gewählt werden. Übliche Verfahren sind in W. A. Ritschel, Die Tablette, 2. Aufl. Kap. 3, Mischen und Zerkleinern, Editio Cantor Verlag Aulendorf 2002 beschrieben. Die gewünschte Korngröße kann beispielsweise durch Klassieren, z.B. Mahlen und/oder Sieben, eingestellt werden.

Aufgrund der geringen Wasseraufnahme ist das erfindungsgemäße Topiramat für die Herstellung pharmazeutischer Formulierungen, insbesondere in Form von Tabletten oder Kapseln, geeignet. Die vorliegende Erfindung betrifft daher auch pharmazeutische Formulierungen, die Topiramat gemäß der vorstehenden Beschreibung enthalten. In einer besonders bevorzugten Ausführungsform ist die pharmazeutische Formulierung durch Direktverpressung erhältlich, insbesondere handelt es sich um direktverpresste Tabletten.

Zur Herstellung der Tabletten wird der Wirkstoff mit üblichen Hilfsmitteln, die dem Fachmann bekannt sind, vermischt und vorzugsweise direktverpresst. Als Hilfsmittel eignen sich beispielsweise mikrokristalline Cellulose, Magnesiumstearat, Lactose-Monohydrat, Maisstärke, Crospovidon sowie hochdisperses Siliciumdioxid. Die Mengen der einzelnen Bestandteile können vom Fachmann so gewählt werden, dass Tabletten mit einem gewünschten Wirkstoffgehalt erhalten werden. Die Tabletten können auf geeignete Weise mit Überzügen versehen werden, wobei Polymethacrylat- und Hydroxypropylmethylcellulose-haltige Filme besonders bevorzugt sind.

Die durch Direktverpressen erhältlichen erfindungsgemäßen Tablettenformulierungen nehmen weniger Feuchtigkeit auf als handelsübliche Tabletten. Es hat sich gezeigt, dass Topamax 200 mg bzw. Epitomax 100 mg bei 25°C und 90 % r.F. 9 % Wasser aufnehmen. Demgegenüber nehmen erfindungsgemäße Topiramattabletten gemäß nachfolgendem Beispiel 1 unter gleichen Bedingungen nur 5,5 % Wasser auf.

Die vorliegende Erfindung betrifft ferner die Verwendung des beschriebenen Topiramats zur Herstellung einer pharmazeutischen Formulierung sowie Verfahren zur Herstellung entsprechender pharmazeutischer Formulierungen.

Mit den nachfolgenden beispielhaften erfindungsgemäßen Tablettenformulierungen hat sich darüber hinaus gezeigt, dass entgegen der Lehre der WO 01/89445 Topiramat-Tabletten mit einem Gehalt an freiem Wasser von etwa 2 % (Beispiele 1 und 3) bis 3 % (Beispiel 2), d.h. ohne Vortrocknung auf weniger als 1,4 % freies Wasser, selbst bei unverpackter, offener Lagerung über zwei Monate bei 40°C und 75 % r.F. keine der in der WO-Schrift beschriebenen Instabilitäten, wie Verfärbung und Bildung von Sulfationen, aufweisen.

Die vorliegende Erfindung wird nun anhand der nachfolgenden Beispiele, die nicht einschränkend auszulegen sind, näher erläutert.

### Beispiele

Die in folgenden Beispielen angegebenen Rezepturen eignen sich zur Herstellung direktverpresster Tablettenkerne. Die Tablettenkerne können mit einem geeigneten Film überzogen werden. Die Prozentangaben sind in Gew.-%.

**Beispiel 1:**

| | |
|---|---|
| Topiramat | 57,15 % |
| Mikrokristalline Cellulose | 42,35 % |
| Magnesiumstearat | 0,50 % |
| Gewicht | ca. 350 mg |
| Härte | 75 N |
| Zerfallzeit | 15 s |

**Beispiel 2:**

| | |
|---|---|
| Topiramat | 41,70 % |
| Mikrokristalline Cellulose | 57,80 % |
| Magnesiumstearat | 0,50 % |
| Gewicht | ca. 480 mg |
| Härte | 160 N |
| Zerfallzeit | 15 s |

**Beispiel 3:**

| | |
|---|---|
| Topiramat | 41,50 % |
| Mikrokristalline Cellulose | 16,50 % |
| Lactose-Monohydrat / Maisstärke (85:15) | 35,50 % |
| Crospovidon | 5,00 % |
| Hochdisperses Siliziumdioxid | 1,00 % |
| Magnesiumstearat | 0,50 % |

Es wurden zwei Chargen mit folgenden Eigenschaften hergestellt:
a.)

| | |
|---|---|
| Gewicht | ca. 120 mg |
| Härte | 65 N |
| Zerfallzeit | 1 0 s |

b.)

| | |
|---|---|
| Gewicht | ca. 480 mg |
| Härte | 190 N |
| Zerfallzeit | 39 s |

Die nach den vorstehenden Beispielen hergestellten Tabletten wiesen einen Gehalt an freiem Wasser von etwa 2 % (Beispiele 1 und 3) bzw. 3% (Beispiel 2) auf. Selbst bei unverpackter, offener Lagerung über zwei Monate bei 40°C und 75 % r.F. wiesen sie keine Instabilitäten im Hinblick auf Verfärbung oder Bildung von Sulfationen auf.

## Patentansprüche

1. Topiramat **dadurch gekennzeichnet, dass** es in hoch reiner Form vorliegt und mindestens 90 % der Teilchen eine Korngröße von ≤ 250 µm aufweisen.

2. Topiramat nach Anspruch 1, das höchstens 0,5 Gew.-% Verunreinigungen enthält.

3. Topiramat nach Anspruch 2, das höchstens 0,2 Gew.-% Verunreinigungen enthält.

4. Topiramat nach einem der vorhergehenden Ansprüche, wobei mindestens 90 % der Teilchen eine Korngröße von ≤ 200 µm aufweisen.

5. Topiramat nach einem der vorhergehenden Ansprüche, das eine mittlere Korngröße von ≤ 150 µm, vorzugsweise ≤ 120 µm und besonders bevorzugt im Bereich von 30-80 µm aufweist.

6. Topiramat **dadurch gekennzeichnet, dass** es im wesentlichen nicht hygroskopisch ist.

7. Topiramat nach einem der vorhergehenden Ansprüche, das bei 25°C und 90 % relativer Feuchtigkeit < 0,1 % Wasser bezogen auf das Gewicht des Topiramats vor der Wasseraufnahme aufnimmt.

8. Topiramat nach einem der vorhergehenden Ansprüche, das nach Lagerung für mindestens einen Monat bei 80°C keine farblichen Veränderungen zeigt.

9. Pharmazeutische Formulierung, enthaltend Topiramat gemäß einem der vorhergehenden Ansprüche.

10. Pharmazeutische Formulierung nach Anspruch 9, die in Form einer Tablette oder Kapsel vorliegt.

11. Pharmazeutische Formulierung nach Anspruch 9 oder 10, die durch Direktverpressung erhältlich ist.

12. Verwendung von Topiramat nach einem der Ansprüche 1-8 zur Herstellung einer pharmazeutischen Formulierung.

13. Verfahren zur Herstellung einer pharmazeutischen Formulierung, wobei Topiramat nach einem der Ansprüche 1-8 mit pharmazeutisch verträglichen Hilfsmitteln gemischt und zu der pharmazeutischen Formulierung verarbeitet wird.

14. Verfahren nach Anspruch 13, wobei es sich bei der pharmazeutischen Formulierung um eine Tablette oder Kapsel handelt.

15. Verfahren nach Anspruch 13 oder 14, wobei die pharmazeutische Formulierung durch Direktverpressung erhalten wird.
